(19) Europäisches Patentamt
European Patent Office
Office européen des brevets

(11) Veröffentlichungsnummer: **0 356 843 A2**

(12) **EUROPÄISCHE PATENTANMELDUNG**

(21) Anmeldenummer: **89115313.2**

(22) Anmeldetag: **19.08.89**

(51) Int. Cl.[5]: **C07D 213/50 , C07D 231/12 , C07D 233/54**

(30) Priorität: **27.08.88 DE 3829144**

(43) Veröffentlichungstag der Anmeldung: **07.03.90 Patentblatt 90/10**

(84) Benannte Vertragsstaaten: **BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Eggersdorfer, Manfred, Dr.**
**Hannongstrasse 18**
**D-6710 Frankenthal(DE)**
Erfinder: **Brenner, Karl**
**Riedsaumstrasse 40**
**D-6700 Ludwigshafen(DE)**
Erfinder: **Henkelmann, Jochem, Dr.**
**Volkerstrasse 26**
**D-6140 Bensheim(DE)**
Erfinder: **Goetz, Norbert, Dr.**
**Schoefferstrasse 25**
**D-6520 Worms 1(DE)**
Erfinder: **Kuekenhoehner, Thomas, Dr.**
**Seidelstrasse 2**
**D-6710 Frankenthal(DE)**

(54) **Verfahren zur Herstellung von heteroaromatischen Aldehyden und Ketonen.**

(57) Herstellung von heteroaromatischen Aldehyden und Ketonen der allgemeinen Formel I

$$Ar_{het}-\overset{\overset{O}{\|}}{C}-R^1 \qquad I$$

($Ar_{het}$: 5- oder 6-gliedriger thermostabiler Heteroarylrest mit mindestens einem N-Atom; $R^1$ Wasserstoff oder $C_1$-$C_{20}$-Alkylrest) durch oxidative Dehydrierung der entsprechenden Alkohole II

$$Ar_{het}-\overset{\overset{OH}{|}}{CH}-R_1 \qquad II$$

mit Sauerstoff oder Sauerstoff enthaltenden Gasen bei 300 °C - 700 °C in der Gasphase in Gegenwart eines Katalysators.

EP 0 356 843 A2

## Verfahren zur Herstellung von heteroaromatischen Aldehyden und Ketonen

Die vorliegende Erfindung betrifft ein neues Verfahren zur Herstellung von heteroaromatischen Aldehyden und Ketonen der allgemeinen Formel I

$$Ar_{het}-\overset{\overset{O}{\|}}{C}-R^1 \qquad I$$

in der $Ar_{het}$ einen 5- oder 6-gliedrigen thermostabilen Heteroarylrest mit mindestens einem Stickstoffatom und $R^1$ Wasserstoff oder einen $C_1$-$C_{20}$-Alkylrest bedeuten, durch oxidative Dehydrierung der entsprechenden Alkohole II

$$Ar_{het}-\overset{\overset{OH}{|}}{CH}-R_1 \qquad II$$

Verbindungen des Typs (I) sind nach den bisher bekannt gewordenen Verfahren wirtschaftlich nur schlecht zugänglich.

So werden die Pyridylalkylketone durch Oxidation der entsprechenden Alkohole mit Chromtrioxid/Schwefelsäure (V. Deljac et al, Acta Pharm. Jugosl. 32, 267-74 (1982); Chem. Abstr. 98, 174 241t) oder mit Mangandioxid (US-A 4 098 908) hergestellt. Im Falle der Pyridinaldehyde finden Oxidationsmittel wie Selendioxid, Bleitetraacetat oder Mangandioxid Verwendung (vgl. Ullmanns Encyklopädie der Technischen Chemie, 3. Auflage, Bd. 14, 1963, S. 478).

Der Nachteil dieser Verfahren ist jedoch, daß sie wegen der physiologisch nicht unbedenklichen Oxidationsmittel einen erheblichen verfahrenstechnischen Aufwand erfordern und außerdem sind die Ausbeuten und Selektivitäten meist unbefriedigend.

Weiterhin ist bekannt, aliphatische oder/und isoaromatische Alkohole in der Gasphase katalytisch oxidativ zu dehydrieren. So betrifft die US-A 2 042 220 die oxidative Dehydrierung von 3-Methyl-3-buten-1-ol mit Sauerstoff bei 360-550 °C in Gegenwart von Metallkatalysatoren. Ähnliche Reaktionen sind in der DE-B 2 715 209 und der EP-B 55 354 beschrieben.

Nachteilig an diesem Verfahren ist jedoch, daß gute Selektivitäten nur erreicht werden können, wenn bestimmte Katalysatorkorngrößen und -korngrößenverteilungen in bestimmter Anordnung dieser Katalysatorteilchen eingehalten werden, was aufwendige Verfahren bei der Reaktorfüllung voraussetzt.

Nach der FR-A 2 231 650 wird die oxidative Dehydrierung von aliphatischen und isoaromatischen Alkoholen mit Luftsauerstoff zu Aldehyden und Ketonen in Gegenwart massiver Goldkatalysatoren vorgenommen. Die Selektivität des Verfahrens ist zwar gut, die Umsätze sind jedoch meistens unbefriedigend.

Der Erfindung lag die Aufgabe zugrunde, heteroaromatische Aldehyde und Ketone auf einfachere und wirtschaftlichere Weise zugänglich zu machen als bisher.

Demgemäß wurde ein Verfahren zur Herstellung von heteroaromatischen Aldehyden und Ketonen der allgemeinen Formel I

$$Ar_{het}-\overset{\overset{O}{\|}}{C}-R_1 \qquad I$$

in der $Ar_{het}$ einen 5- oder 6-gliedrigen thermostabilen Heteroarylrest mit mindestens einem Stickstoffatom und $R^1$ Wasserstoff oder einen $C_1$-$C_{20}$-Alkylrest bedeuten, durch oxidative Dehydrierung der entsprechenden Alkohole II

$$Ar_{het}-\overset{\overset{OH}{|}}{CH}-R^1 \qquad II$$

gefunden, welches dadurch gekennzeichnet ist, daß man die oxidative Dehydrierung mit Sauerstoff oder einem Sauerstoff enthaltenden Gas in der Gasphase in Gegenwart eines Katalysators aus Kupfer, Silber und/oder Gold bei Temperaturen von 300 °C-700 °C durchführt.

Ferner wurde gefunden, daß es vorteilhaft ist, die Reaktion bei einem Druck von 100 mbar bis 700 mbar auszuführen.

Definitionsgemäß enthalten die Reste $Ar_{het}$ in den Ausgangsverbindungen II und damit in den Verfahrensprodukten I mindestens ein Stickstoffatom im Ring, d.h. der Ring kann auch noch weitere Heteroatome wie Stickstoff, Sauerstoff und Schwefel enthalten. Die Maßgabe, der Rest $Ar_{het}$ solle thermostabil sein, bedeutet, daß er sich unter den Reaktionsbedingungen oberhalb 300 °C nicht zersetzt.

Als thermostabile Grundkörper seien beispielsweise folgende Heteroaromaten genannt:

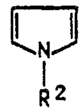

Pyrrol

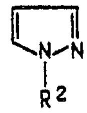

Pyrazol

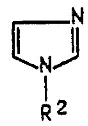

Imidazol

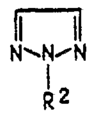

1,2,3-Triazol

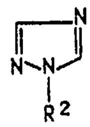

1,2,4-Triazol

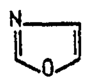

Oxazol

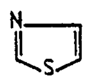

Thiazol

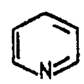

Pyridin

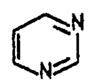

Pyrimidin

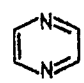

Pyrazin

wobei $R^2$ Wasserstoff oder vorzugsweise eine $C_1$-$C_4$-Alkylgruppe, insbesondere die Methylgruppe, oder die Benzylgruppe bedeutet.

Im Hinblick auf die Verfahrensprodukte, die ihrerseits als Zwischenprodukte für Arznei- und Pflanzenschutzmittel dienen, kommt den Verbindungen der Pyridin- und der Pyrazol-Reihe besondere Bedeutung zu.

Die Hydroxyalkylgruppe kann an ein beliebiges C-Atom des Rings gebunden sein, und die übrigen C-Atome des Rings können weitere Substituenten tragen, die sich unter den Reaktionsbedingungen inert verhalten, beispielsweise $C_1$-$C_4$-Alkyl- und Alkoxygruppen.

$R^1$ bedeutet vorzugsweise Wasserstoff oder einen $C_1$ - $C_{20}$-Alkylrest, besonders die Hexyl- und die 4-Methyl-pentylgruppe.

Die Alkohole II sind bekannt oder nach bekannten Methoden erhältlich. So werden z.B. die besonders wichtigen sekundären Pyridylalkohole durch Addition von Alkylmagnesiumbromiden an Pyridinaldehyd hergestellt (vgl. V. Deljac et al, Acta Pharm. Jugosl., 32, 267-74 (1982)).

Als wichtige Vorstufen der Zwischenprodukte I für die Herstellung von Pharmaka und Pflanzenschutzmitteln dienen z.B. folgende Alkohole II:

3-Hydroxymethyl-pyrazol
3-Hydroxymethyl-2-methyl-pyrazol
3-(1-Hydroxypentyl)-2-methyl-pyrazol
2-Hydroxymethyl-2-methyl-imidazol

3-(1-Hydroxyhexyl)-2-methyl-imidazol
2-Hydroxymethyl-pyridin
3-Hydroxymethyl-pyridin
4-Hydroxymethyl-pyridin
3-(1-Hydroxypentyl)-pyridin
3-(1-Hydroxy-4-methyl-pentyl)-pyridin
3-(2-Ethyl-1-hydroxypentyl)-pyridin
3-(1-Hydroxyxhexyl)-pyridin.

Die Menge des für die oxidative Dehydrierung benötigten Sauerstoffs ist nicht kritisch, sollte aber für einen möglichst vollständigen Umsatz mindestens 0,5 mol $O_2$ pro Mol II betragen. Kleinere Mengen, etwa 0,2 bis 0,5 mol $O_2$ pro Mol II, können vorteilhaft sein, wenn man sich zur Selektivitätserhöhung mit einem geringeren Umsatz begnügt, und ab 4 mol $O_2$/mol II kann im allgemeinen mit einem nennenswerten Einfluß auf Umsatz und Reaktionsgeschwindigkeit nicht mehr gerechnet werden.

In aller Regel empfiehlt es sich, ein Inertgas wie Argon, Stickstoff und/oder Kohlendioxid mitzuverwenden, und zwar vorzugsweise im Molverhältnis Inertgas zu $O_2$ von 2:1 bis 20:1. Die verfahrenstechnisch einfachste und meist auch wirtschaftlichste Arbeitsweise besteht in der Verwendung von Luft als Oxidationsmittel. Wasserdampf als Inertgas oder als Bestandteil eines Inertgasgemisches hat in manchen Fällen einen günstigen Einfluß auf die Selektivität bezüglich des gewünschten Verfahrensprodukts.

Als Katalysatoren sind Kupfer, Silber und/oder Gold geeignet. Diese Metalle können als solche, vorzugsweise zerkleinert oder in kristalliner Form oder in anderen großen Oberflächen bildenden Formen, beispielsweise als Drahtnetze oder Formlinge, eingesetzt werden. Ebenfalls kann es vorteilhaft sein, diese Metalle auf Trägermaterialien (z.B. Kieselgel, Aluminiumoxid Tonerde u.ä.) abgeschieden, also in Form von Trägerkatalysatoren, zu verwenden. Im Falle von Trägerkatalysatoren beträgt die Schichtdicke der aktiven Masse vorzugsweise 5 bis 25 µm. Der Durchmesser der Trägerkatalysatorpartikel liegt vorzugsweise zwischen 1,6 und 2,0 mm. In erster Linie kommen kugelförmige oder annähernd kugelförmige Trägerpartikel in Betracht.

Im Falle von Massivkatalysatoren werden elektrolytisch abgeschiedene Kristalle mit einer Korngröße von 0,1 bis 10 mm bevorzugt.

Man kann die Reaktion in einem Wirbelbett ausführen, wobei man einen sehr feinteiligen Katalysator verwendet, jedoch ordnet man den Katalysator vorzugsweise in einem Rohr- oder Rohrbündelreaktor in Form eines Festbettes an, dessen Höhe etwa 5 bis 30, vorzugsweise 10 bis 20 cm beträgt.

Die bevorzugten Reaktionstemperaturen liegen zwischen 450 und 600 °C.

Die Umsetzung gelingt umso besser, je geringer der Partialdruck des gasförmigen Alkohols II ist. Da mit sinkendem Partialdruck jedoch auch der Umsatz abnimmt, wird man im allgemeinen keinen Partialdruck unter 10 mbar wählen. Die obere Grenze für den Partialdruck, bis zu welcher im allgemeinen optimale Ergebnisse erzielt werden, liegt etwa bei 250 mbar.

Der bevorzugte Partialdruckbereich entspricht etwa einem Gesamtdruckbereich von 100 bis 700 mbar, insbesondere 400 - 600 mbar, d.h., es ist zweckmäßig, die oxidative Dehydrierung unter einem Druck aus diesem Bereich vorzunehmen. Möchte man, was verfahrenstechnisch einfacher ist, bei höherem Druck, insbesondere Normaldruck, arbeiten, so lassen sich die Empfehlungen für den Partialdruck auch durch Mitverwendung entsprechend größerer Inertgasmengen verwirklichen.

Es ist zweckmäßig, den Alkohol II im Inertgasstrom zu verdampfen, das Gasgemisch auf eine Temperatur, die etwa 100 bis 250 °C unterhalb der Reaktionstemperatur liegt, zu erhitzen und sodann durch die Katalysatorschicht zu leiten, wobei die Verweilzeit in dieser Schicht vorzugsweise 0,001 bis 5, insbesondere 0,01 bis 0,2 sec beträgt.

Das heiße Gasgemisch wird nach der Umsetzung durch Einsprühen von Lösungsmitteln abgekühlt und kondensiert. Als Lösungsmittel eignen sich Kohlenwasserstoffe wie Hexan und Toluol oder Ether wie Methyl.tert.-butylether oder Dimethylformamid.

Im übrigen bietet das erfindungsgemäße Verfahren keine verfahrenstechnischen Besonderheiten. Das gleiche gilt für die Isolierung der Verfahrensprodukte I.

Die Standzeit des Katalysators beträgt bei konstanter Selektivität und Aktivität mindestens drei Monate.

Man erhält die heteroaromatischen Aldehyde und Ketone I, die u.a. wertvolle Zwischenprodukte für die Herstellung von Pharmaka und Pflanzenschutzmitteln sind, auf einfache Weise und in bemerkenswert guter Ausbeute.

Beispiel 1

Herstellung von 3-(1-oxo-hexyl)-pyridin

Stündlich wurde ein Gasgemisch aus 33 g (0, 19 mol) 3-(1-Hydroxyhexyl)-pyridin, 30 Nl Luft und 15 Nl Stickstoff auf 220 °C erhitzt und von oben nach unten durch eine in einem Rohr angeordnete Katalysatorschicht von 10 cm Höhe und 1,2 cm Durchmesser geleitet. Der Katalysator bestand aus Silberkristallen von 0,4 bis 0,7 mm mittlerem Durchmesser und die Reaktionstemperatur betrug 450 °C.

Das gasförmige Reaktionsgemisch wurde auf 20 - 25 °C abgekühlt und in Dimethylformamid aufgenommen. Die übliche Aufarbeitung lieferte das Verfahrensprodukt in einer Selektivität von 78 %, bezogen auf einen Umsatz von fast 99 %.

Beispiel 2

Herstellung von 3-(4-Methyl-1-oxo-pentyl)-pyridin

Analog Beispiel 1 wurden 33 g (0,17 mol) 3-(1-Hydroxy-4-methyl-pentyl)-pyridin bei einer Temperatur von 470 °C umgesetzt. Nach der üblichen Aufarbeitung wurde das Verfahrensprodukt mit einer Selektivität von 75,8 % bei einem Umsatz von 96,7 % erhalten.

Beispiel 3

Herstellung von 3-(2-Ethyl-1oxo-pentyl)-pyridin

Analog Beispiel 1 wurden 33 g (0,15 mol) 3-(1-Hydroxy-4-methyl-pentyl)-pyridin bei einer Temperatur von 450 °C umgesetzt. Nach der üblichen Aufarbeitung wurde das Verfahrensprodukt mit einer Selektivität von 73 % bei einem Umsatz von 84,9 % erhalten.

**Ansprüche**

1. Verfahren zur Herstellung von heteroaromatischen Aldehyden und Ketonen der allgemeinen Formel I

$$Ar_{het}-\overset{O}{\overset{\|}{C}}-R^1 \qquad I$$

in der $Ar_{het}$ einen 5- oder 6-gliedrigen thermostabilen Heteroarylrest mit mindestens einem Stickstoffatom und $R^1$ Wasserstoff oder einen $C_1$-$C_{20}$-Alkylrest bedeuten, durch oxidative Dehydrierung der entsprechenden Alkohole II

$$Ar_{het}-\overset{OH}{\overset{|}{CH}}-R_1 \qquad II$$

dadurch gekennzeichnet, daß man die Reaktion in der Gasphase mit Sauerstoff oder einem Sauerstoff enthaltenden Gas in Gegenwart eines Katalysators aus Kupfer und/oder Silber und/oder Gold bei Temperaturen von 300 °C - 700 °C ausführt.

2. Verfahren nach Anspruch 1, dadurch gekennzeichnet, daß man die Reaktion bei 100 - 700 mbar ausführt.

3. Verfahren nach den Ansprüchen 1 oder 2, dadurch gekennzeichnet, daß $Ar_{het}$ in den Verbindungen I bzw. II ein substituierter oder unsubstituierter Pyridinring ist.